(19) **Europäisches Patentamt**
**European Patent Office**
**Office européen des brevets**

(11) **EP 3 882 603 B1**

(12) **EUROPEAN PATENT SPECIFICATION**

(45) Date of publication and mention
of the grant of the patent:
**07.01.2026 Bulletin 2026/02**

(21) Application number: **19883640.5**

(22) Date of filing: **06.11.2019**

(51) International Patent Classification (IPC):
***G01N 15/14*** *(2024.01)* ***G01N 21/64*** *(2006.01)*

(52) Cooperative Patent Classification (CPC):
**G01N 15/1459; G01N 15/1429; G06F 18/10;**
**G06F 18/217; G06F 18/232; G06F 18/40;**
G01N 2015/1006; G01N 2015/1402;
G01N 2015/1488

(86) International application number:
**PCT/JP2019/043368**

(87) International publication number:
**WO 2020/100667 (22.05.2020 Gazette 2020/21)**

(54) **INFORMATION PROCESSING DEVICE, INFORMATION PROCESSING METHOD, AND COMPUTER PROGRAM**

INFORMATIONSVERARBEITUNGSVORRICHTUNG, INFORMATIONSVERARBEITUNGSVERFAHREN UND COMPUTERPROGRAMM

DISPOSITIF DE TRAITEMENT D'INFORMATIONS, PROCÉDÉ DE TRAITEMENT D'INFORMATIONS ET PROGRAMME INFORMATIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.11.2018 JP 2018215619**

(43) Date of publication of application:
**22.09.2021 Bulletin 2021/38**

(73) Proprietor: **Sony Group Corporation Minato-ku, Tokyo 108-0075 (JP)**

(72) Inventors:
• **YAMANE, Kenji Tokyo 108-0075 (JP)**
• **ENOKI, Junichiro Tokyo 108-0075 (JP)**
• **MURATA, Rei Tokyo 108-0075 (JP)**

(74) Representative: **D Young & Co LLP 3 Noble Street London EC2V 7BQ (GB)**

(56) References cited:
WO-A1-2017/191699 JP-A- 2010 227 326
JP-A- 2015 135 318 JP-A- 2017 505 901
US-A1- 2019 137 383

• **DANIEL JIMENEZ-CARRETERO ET AL: "Flow Cytometry Data Preparation Guidelines for Improved Automated Phenotypic Analysis", THE JOURNAL OF IMMUNOLOGY, vol. 200, no. 10, 7 May 2018 (2018-05-07), US, pages 3319 - 3331, XP055666227, ISSN: 0022-1767, DOI: 10.4049/ jimmunol.1800446**
• **ZARE HABIL ET AL: "Data reduction for spectral clustering to analyze high throughput flow cytometry data", BMC BIOINFORMATICS, BIOMED CENTRAL , LONDON, GB, vol. 11, no. 1, 28 July 2010 (2010-07-28), pages 403, XP021071730, ISSN: 1471-2105, DOI: 10.1186/ 1471-2105-11-403**
• **KIERAN O'NEILL ET AL: "Flow Cytometry Bioinformatics", PLOS COMPUTATIONAL BIOLOGY, vol. 9, no. 12, 5 December 2013 (2013-12-05), XP055665872, DOI: 10.1371/ journal.pcbi.1003365**

**Description**

Field

The present disclosure relates to an information processing apparatus, an information processing method, and a computer program.

Background

In the medical and biological fields and the like, it is common to use a flow cytometer to rapidly measure characteristics of each of a large amount of particles. The flow cytometer is a device that irradiates particles, such as cells or beads, which flow in a flow cell with laser beams, detects fluorescence, scattered light, or the like emitted by the particles, and optically measures characteristics of each of the particles.

For example, Patent Literature 1 listed below discloses a technique of detecting, in fluorescence detection by a flow cytometer (microparticle measurement device), intensity of light in a continuous wavelength band as a fluorescence spectrum. In the microparticle measurement device disclosed in Patent Literature 1, a spectral element, such as a prism or a grating, is used to disperse fluorescence emitted from the microparticle into a spectrum, and a light-receiving element array, in which a plurality of light-receiving elements compatible with different detection wavelength bands are arrayed, detects the dispersed fluorescence.

Further, as the flow cytometer, a spectrum system that is able to detect each fluorescence spectrum as a form and use every piece of fluorescence information as analysis information is present, in contrast to a filter system that detects a part of wavelength regions of fluorescence. Citation List

Patent Literature:

Patent Literature 1: Japanese Patent No. 5772425
Patent Literature 2: WO 2017/191699 A1

Non-Patent Literature (NPL):

NPL 1: Daniel Jimenez-Carretero et al., "Flow Cytometry Data Preparation Guidelines for Improved Automated Phenotypic Analysis", The Journal of Immunology, Vol. 200, No. 10 7 May 2018, pages 3319-3331
NPL 2: Zare Habil et al., "Data Reduction for Spectral Clustering to Analyze High Throughput Flow Cytomtery Data", BMC Bioinformatics, Biomed Central, London Vol. 11, No. 1, 28 July 2010, page 403
NPL 3: Kieran O'Neill et al., "Flow Cytometry Bioinformatics", PLoS Computational Biology, Vol. 9, No. 12, 5 December 2013

Summary

Technical Problem

However, the flow cytometer of the spectrum system obtains a measured value for each of wavelengths, so that a feature amount acquired from a single cell increases. Therefore, when data clustering is performed, problems with an increase in a processing time and the curse of dimensionality are likely to occur.

To cope with this, in the present disclosure, an information processing apparatus, an information processing method, and a computer program that are novel, modified, and able to reduce a processing time at the time of analysis of information obtained from a measurement device of a spectrum system are proposed.

Solution to Problem

According to the present disclosure, an information processing apparatus comprising: a compression unit that performs a compression process on a data amount of spectral data that is measured when a particle is irradiated with a laser beam; an acquisition unit that acquires a result of a clustering process performed on the compressed spectral data; and an output unit that outputs the result of the clustering process acquired by the acquisition unit is presented.

Furthermore, according to the present disclosure, an information processing method implemented by a processor, the information processing method comprising: performing a compression process on a data amount of spectral data that is measured when a particle is irradiated with a laser beam; acquiring a result of a clustering process performed on the compressed spectral data; and outputting the acquired result of the clustering process is presented.

Moreover, according to the present disclosure, a computer program that causes a computer to execute: performing a compression process on a data amount of spectral data that is measured when a particle is irradiated with a laser beam; acquiring a result of a clustering process performed on the compressed spectral data; and outputting the acquired result of the clustering process is presented.

Advantageous Effects of Invention

As described above, according to the present disclosure, it is possible to provide an information processing apparatus, an information processing method, and a computer program that are novel, modified, and able to reduce a processing time at the time of analysis of information obtained from a measurement device of a spectrum system.

Further, the effects described above are not limitative. That is, with or in the place of the above effects, any of the effects described in this specification or other effects that can be recognized from this specification may be achieved.

Brief Description of Drawings

FIG. 1 is a block diagram for explaining an information processing system according to the present embodiment.
FIG. 2 is a block diagram for explaining a flow cytometer included in the information processing system.
FIG. 3 is a block diagram for explaining the flow cytometer included in the information processing system.
FIG. 4 is an explanatory diagram illustrating a functional configuration example of an information processing apparatus according to the present embodiment.
FIG. 5 is a flow diagram illustrating an operation example of the information processing system according to the present embodiment.
FIG. 6 is an explanatory diagram illustrating an example of how an analysis result is drawn by the information processing apparatus.
FIG. 7 is an explanatory diagram illustrating an example of how the analysis result is drawn by the information processing apparatus.
FIG. 8 is an explanatory diagram illustrating an example of how the analysis result is drawn by the information processing apparatus.
FIG. 9 is an explanatory diagram illustrating an example of how the analysis result is drawn by the information processing apparatus.
FIG. 10 is a diagram for explaining an example of visualization by decimation using a cluster label.
FIG. 11 is a flow diagram illustrating an operation example of the information processing apparatus according to the present embodiment.
FIG. 12 is an explanatory diagram illustrating an example of a user interface provided by the information processing apparatus according to the present embodiment.
FIG. 13 is a flow diagram illustrating an operation example of the information processing apparatus according to the present embodiment.
FIG. 14 is a flow diagram illustrating an operation example of the information processing apparatus according to the present embodiment.
FIG. 15 is a flow diagram illustrating an operation example of the information processing system according to the present embodiment.
FIG. 16 is a block diagram illustrating one example of a hardware configuration of the information processing apparatus according to the present embodiment.

Description of Embodiments

Preferred embodiments of the present disclosure will be described in detail below with reference to the accompanying drawings. Meanwhile, in the present specification and drawings, structural elements having substantially the same functions and configurations are denoted by the same reference symbols, and repeated explanation will be omitted.

In addition, hereinafter, explanation will be given in the following order.

1. Embodiment of present disclosure

    1.1. Background
    1.2. Configuration Example
    1.3. Operation Example

2. Hardware Configuration
3. Conclusion

1. Embodiment of present disclosure

1.1. Background

Before explaining embodiments of the present disclosure in detail, a background of the embodiments of the present disclosure will be described

As described above, in the medical and biological fields and the like, it is common to use a flow cytometer to rapidly measure characteristics of each of a large amount of particles. The flow cytometer is a device that irradiates particles, such as cells or beads, which flow in a flow cell with laser beams, detects fluorescence, scattered light, or the like emitted by the particles, and optically measures characteristics of each of the particles.

In recent years, the flow cytometer is able to obtain a lot of information by measuring fluorescence signals using a large number of dyes at once by performing staining with a large number of colors. In contrast, the limitations of analysis using conventional manual gating have been pointed out. For example, patterns of two-dimensional plot for n kinds of fluorescent dyes are represented by $_nC_2$. In other words, there are 15 patterns for six colors, and the number of patterns increases to 190 for 20 colors. Therefore, automatic analysis using clustering is expected to serve as an alternative to the manual gating.

Furthermore, as the flow cytometer, a spectrum system that is able to detect each fluorescence spectrum as a form and use every piece of fluorescence information as information is present, in contrast to a filter system that detects a part of wavelength regions of fluorescence. In this system, a measured value is obtained for each of the wavelengths, so that a feature amount acquired from a single cell increases. Therefore, in clustering, an increase in a processing time and the curse of dimensionality are likely to occur.

Moreover, in the clustering that is commonly used in a current flow cytometer, a certain cell is included in any of clusters, so that data that is located at a boundary of two or more clusters and that is difficult to be distinguished is also included in any of the clusters. Furthermore, with the improvement of a measurement speed of the flow cytometer, the number of pieces of data to be analyzed is increasing, and the increase in the number of pieces of data leads to an increase in a drawing time in data analysis. Furthermore, when a cluster generated by a user additionally analyzed, an analysis time increases simply because of an increase in the number of target clusters in addition to an increase in the drawing time.

Therefore, in view of the foregoing points, the disclosers of the present application earnestly examined a technology capable of reducing a processing time at the time of clustering information obtained from a flow cytometer and reducing a drawing time of a clustering result. As a result, the disclosers of the present application conceived the technology capable of reducing a processing time at the time of clustering information obtained from a flow cytometer and reducing a drawing time of a clustering result as described below.

1.2. Configuration Example

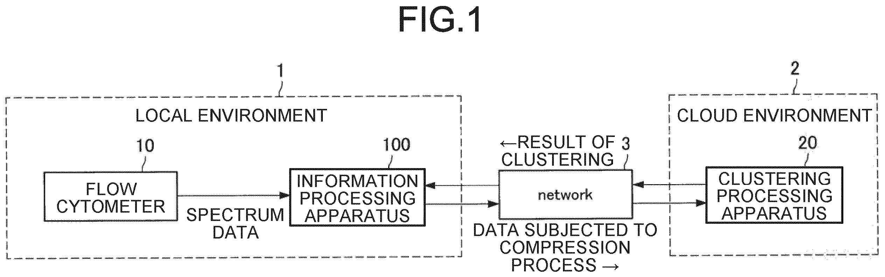

Next, with reference to FIG. 1, an overview of an information processing system according to one embodiment of the present disclosure will be described. FIG. 1 is a block diagram for explaining the information processing system according to the present embodiment.

As illustrated in FIG. 1, the information processing system according to the present embodiment includes a flow cytometer 10 and an information processing apparatus 100 that are arranged in a local environment 1, and further includes a clustering processing apparatus 20 that is arranged in a cloud environment 2. The local environment 1 and the cloud environment 2 are connected to each other via a network 3. The network 3 may be, for example, the Internet, an intranet, or other networks.

The flow cytometer 10 acquires measured data from a measurement sample. It is preferable that the measured data is data including a spectrum system. The information processing apparatus 100 performs analysis using the measured data acquired by the flow cytometer 10.

The flow cytometer 10 irradiates the measurement sample with laser light, and measures fluorescence, phosphorescence, or scattered light emitted from the measurement sample. It is sufficient for the flow cytometer 10 to measure at least one of the fluorescence, the phosphorescence, and the scattered light emitted from the measurement sample. The flow cytometer 10 may measure an absorption spectrum of the measurement sample instead of or in addition to the fluorescence, the phosphorescence, and the scattered light. Meanwhile, in the following, detailed explanation will be given based on the assumption that the flow cytometer 10 measures at least fluorescence spectra of measurement samples S.

The information processing apparatus 100 determines characteristics or the like of the measurement sample by analyzing the measured data of the measurement sample acquired by the flow cytometer 10. A detailed configuration of the information processing apparatus 100 will be described later. In FIG. 1, a case in which the information processing apparatus 100 and the flow cytometer 10 are arranged as separate devices is illustrated, but the technology according to the present disclosure is not limited to this example. Each of the functions of the information processing apparatus 100 may be mounted on a computer that controls operation of the flow cytometer 10, or may be mounted on an arbitrary computer that is arranged inside a casing of the flow cytometer 10.

The measurement sample measured by the flow cytometer 10 may be a biologically-derived particle, such as a cell, a microorganism, and a biologically relevant particle. For example, the cell may be an animal cell (for example, a blood corpuscle cell or the like), a plant cell, or the like. For example, the microorganism may be bacteria, such as colon bacillus, viruses, such as tobacco mosaic virus, fungi, such as yeast, and the like. The biologically relevant particle may be a particle, such as chromosome, liposome, mitochondria, or various kinds of organelle (cell organelle), that makes up a cell. Meanwhile, the biologically relevant particle may include nucleic acid, protein, lipid, sugar chain, and biologically relevant polymer molecule, such as a complex of nucleic acid, protein, lipid, and sugar chain. The biologically-derived particle may have either spherical shape or a non-spherical shape, and a size and mass thereof are not specifically limited.

Further, the measurement sample may be an industrially synthesized particle, such as a latex particle, a gel particle, or an industrial particle. For example, the industrially synthesized particle may be a particle synthesized with an organic resin material, such as polystyrene and polymethylmethacrylate, an inorganic material, such as glass, silica, and a magnetic body, or metal, such as gold colloid and aluminum. The industrially synthesized particle may similarly have either a spherical shape or a non-spherical shape, and a size and mass thereof are not specifically limited.

The measurement sample may be labeled (stained) by one or more fluorescent dyes before measurement of a fluorescence spectrum. The labeling of the measurement sample using the fluorescent dyes may be performed by a well-known method. Specifically, if the measurement sample is a cell, it is possible to label a measurement target cell by a fluorescent dye by mixing a fluorescence-labeled antibody that selectively binds to an antigen present on a surface of the cell with the measurement target cell, and causing the fluorescence-labeled antibody to bind to the antigen present on the surface of the cell. Alternatively, it is possible to label the measurement target cell by a fluorescent dye by mixing a fluorescent dye that is selectively introduced with respect to a specific cell with the measurement target cell.

The fluorescence-labeled antibody is an antibody that has bound to a fluorescent dye for the purpose of labeling. The fluorescence-labeled antibody may be obtained by causing the antibody to directly bind to the fluorescent dye. Alternatively, the fluorescence-labeled antibody may be obtained by causing the fluorescent dye that has bound to avidin to bind to a biotin-labeled antibody by avidin-biotin reaction. Meanwhile, as the antibody, either a polyclonal antibody or monoclonal antibody may be used.

The fluorescent dye for labeling a cell is not specifically limited, and it is possible to use at least one well-known dye that is used to stain the cell or the like. For example, as the fluorescent dye, it may be possible to use phycoerythrin (PE), fluorescein isothiocyanate (FITC), PE-Cy5, PE-Cy7, PE-Texas Red (registered trademark), allophycocyanin (APC), APC-Cy7, ethidium bromide, propidium iodide, Hoechst (registered trademark) 33258, Hoechst (registered trademark) 33342, DAPI (4', 6-diamidino-2-phenylindole), acridine orange, chromomycin, mithramycin, olivomycin, pyronin Y, thiazole orange, rhodamine 101, isothiocyanate, BCECF, BCECF-AM, C-SNARF-1, C-SNARF-1-AMA, aequorin, Indo-1, Indo-1-AM, Fluo-3, Fluo-3-AM, Fura-2, Fura-2-AM, oxonol, Texas Red (registered trademark), rhodamine 123, 10-N-nonyl-acridine orange, fluorescein, fluorescein diacetate, carboxyfluorescein, carboxyfluorescein diacetate, carboxydichlorofluorescein, carboxydichlorofluorescein diacetate, or the like. Further, it may be possible to use dielectric bodies of the fluorescent dyes as described above.

The information processing apparatus 100 is an apparatus that outputs and/or displays an analysis result of the measurement sample by using a measurement result of the flow cytometer 10. In the present embodiment, the information processing apparatus 100 transfers the measurement result of the flow cytometer 10 to the clustering processing apparatus 20 in the cloud environment 2.

The measurement result of the flow cytometer 10 includes a huge amount of data, and therefore, if the data is transferred as it is to the clustering processing apparatus 20, a transfer time increases. To cope with this, the information processing apparatus 100 performs a predetermined compression process on the measurement result of the flow cytometer 10, and thereafter transfers the measurement result to the clustering processing apparatus 20. Examples of the predetermined compression process performed by the information processing apparatus 100 include dimension compression, such as an unmixing process, and tone compression. The information processing apparatus 100 may control the compression process in a completely automatic manner, or if a parameter, such as the number of dimensions to be compressed, specified by a user is present, the information processing apparatus 100 may perform a process in accordance with the parameter. Further, the information processing apparatus 100 may perform a normalization process before transfer to the clustering processing apparatus 20 to reduce the amount of data. Meanwhile, the information processing apparatus 100 may read data from a file in which spectrum data and data that has been subjected to unmixing in the past are set. The information processing apparatus 100 performs the same flow as described above when re-performing the unmixing using a different parameter. In contrast, in the case of the same parameter, the information processing apparatus 100 reads data from a file as an alternative, without performing unmixing again.

The information processing apparatus 100 may be configured to make one-to-one correspondence between the data subjected to the compression process and the data before subjected to the compression process by using, for example, a data ID or the like.

Further, the information processing apparatus 100 allows a user to select a clustering analysis target by using a two-dimensional plot, a spectrum plot, or the like. When the clustering analysis target it to be selected, original spectrum data that remains in the local environment 1 is used. For example, it may be possible to perform plot using forward scatter (FSC) (forward scattered light) that represents a size of a cell or side scatter (SSC) (side scattered light) that represents complexity of an internal structure of the cell, and perform selection to limit the clustering analysis target to lymphocyte data. This operation may be performed in parallel to the compression process and the operation of transferring data to the cloud environment 2, or may be performed before transferring data to the cloud environment 2. By selecting the clustering analysis target before transferring data to the cloud environment 2, and transferring data of the clustering analysis target prior to other data, it is possible to rapidly start a clustering process in the cloud environment 2. Meanwhile, this step may be omitted when all pieces of data are used as analysis targets for clustering. In any case, the data ID of the clustering analysis target specified by the user is transferred to the cloud environment 2.

The clustering processing apparatus 20 receives the compressed measurement result from the information processing apparatus 100, and performs a clustering process on the received measurement result. The clustering processing apparatus 20 may use any clustering method, such as a k-Means method or hierarchical clustering, or may allow the user to select a clustering method from among a plurality of methods. If the clustering analysis target is specified in the local environment 1, the clustering processing apparatus 20 performs the clustering process on the specified clustering analysis target. The information processing apparatus 100 receives a result of the clustering process performed by the clustering processing apparatus 20, and displays the result of the clustering process if needed.

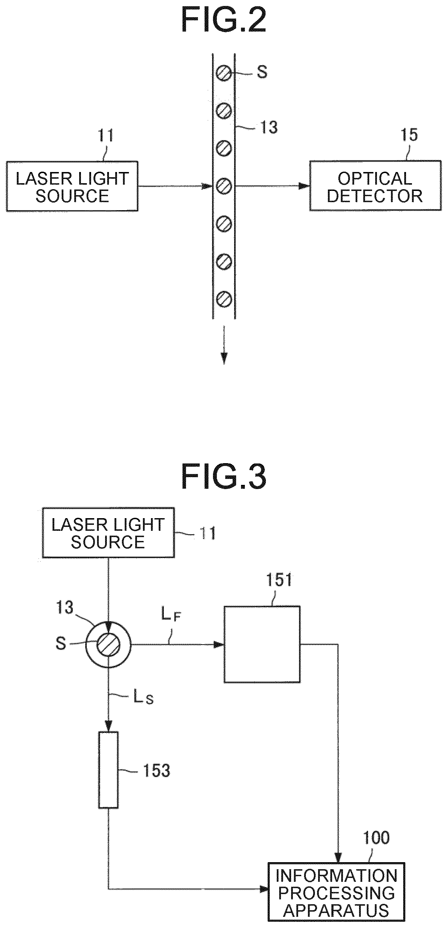

Next, a specific configuration of the flow cytometer 10 will be described with reference to FIG. 2 and FIG. 3. FIG. 2 and FIG. 3 are block diagrams for explaining the flow cytometer 10 included in the information processing system illustrated in FIG. 1.

As illustrated in FIG. 2 and FIG. 3, the flow cytometer 10 includes a laser light source 11 that emits laser light with a wavelength at which the fluorescent dye that labels the measurement samples S can be excited, a flow cell 13 that causes the measurement samples S to flow in one direction, and an optical detector 15 that receives fluorescence, phosphorescence, or scattered light from the measurement samples S irradiated with the laser light.

The laser light source 11 is, for example, a semiconductor laser light source that emits laser light with a predetermined wavelength. It may be possible to arrange the plurality of laser light sources 11. If the plurality of laser light sources 11 are arranged, the laser light sources 11 may apply laser light to the same or different positions in the flow cell 13. However, if the laser light from the plurality of laser light sources 11 is applied to the different positions, it is possible to detect light from the measurement samples S by using the different optical detectors 15, so that it is possible to measure even dyes that emit light with adjacent wavelengths, without color mixture. Meanwhile, the laser light emitted from the laser light source 11 may either pulsed light or continuous light. For example, the laser light source 11 may be a plurality of semiconductor laser light sources that emit laser light with a wavelength of 480 nanometers (nm) and laser light with a wavelength of 640 nm.

The flow cell 13 is a flow passage for causing the plurality of measurement samples S to align and flow in one direction. Specifically, the flow cell 13 flows sheath solution covering the measurement samples S as a laminar flow at a high speed, and causes the plurality of measurement samples S to align and flow in one direction.

The optical detector 15 detects, by photoelectric conversion, the fluorescence, the phosphorescence, and the scattered light that are generated from the measurement sample S irradiated with the laser light.

For example, as illustrated in FIG. 3, the optical detector 15 may include a detector 153 that detects scattered light $L_S$ including forward scattered light and side scattered light from the measurement samples S, and a light-receiving element array 151 that detects fluorescence $L_F$ from the measurement samples S.

The detector 153 may be, for example, a well-known photoelectric conversion element, such as a charge coupled device (CCD), a complementary metal oxide semiconductor (CMOS), or a photodiode. The light-receiving element array 151 may be configured by, for example, arranging a plurality of independent detection channels that detect light in different wavelength bands. Specifically, the light-receiving element array 151 may be a light-receiving element array in which a plurality of photo multiplier tubes (PMTs) or photodiodes that detect different wavelength bands are arranged one-dimensionally, or the like. The light-receiving element array 151 performs photoelectric conversion on fluorescence of the measurement samples S, each of which is dispersed into a spectrum by a spectral element, such as a prism or a grating.

Accordingly, in the flow cytometer 10, the laser light emitted from the laser light source 11 is first applied to each of the measurement samples S that pass through the flow cell 13. By irradiation with the laser light, the measurement samples S emit scattered light and fluorescence (or phosphorescence). Here, the scattered light emitted from the measurement samples S is detected by the detector 153. In contrast, the fluorescence emitted from the measurement sample S is dispersed into a continuous spectrum by a spectral element, and received and detected by the light-receiving element array 151.

With the configuration as described above, the flow cytometer 10 is able to measure the scattered light or the fluorescence from the measurement samples S, and output a measurement result to the information processing apparatus 100.

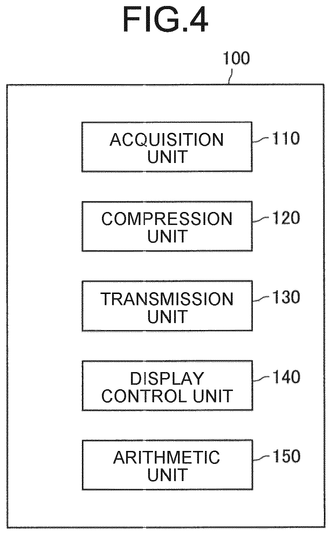

Next, a functional configuration example of the information processing apparatus 100 according to one embodiment of the present disclosure will be described. FIG. 4 is an explanatory diagram illustrating the functional configuration example of the information processing apparatus 100 according to one embodiment of the present disclosure. The functional configuration example of the information processing apparatus 100 according to one embodiment of the present disclosure will be described with reference to FIG. 4.

The information processing apparatus 100 according to one embodiment of the present disclosure includes an acquisition unit 110, a compression unit 120, a transmission unit 130, a display control unit 140, and an arithmetic unit 150.

The acquisition unit 110 acquires the measurement result from the flow cytometer 10. Further, the acquisition unit 110 acquires the result of the clustering process from the cloud environment 2.

The compression unit 120 performs a process of compressing the measurement result obtained by the flow cytometer 10. The compression unit 120 compresses the measurement result by dimension compression, such as an unmixing process, tone compression, or the like. The compression unit 120 may control the compression process in a completely automatic manner, of if a parameter, such as the number of dimensions to be compressed, specified by a user is present, the compression unit 120 may perform a process in accordance with the parameter.

The transmission unit 130 transmits the measurement result compressed by the compression unit 120 to the cloud environment 2. Further, the transmission unit 130 may transmit information for specifying a processing target for clustering in the cloud environment 2 to the cloud environment 2. In the cloud environment 2, a clustering process is performed on the compressed measurement result.

The display control unit 140 performs control of displaying, on an output device, a result of the clustering that is performed in the cloud environment 2 and that is obtained by the acquisition unit 110. The control of displaying the result of the clustering process by the display control unit 140 will be described in detail later.

The arithmetic unit 150 performs arithmetic processing on the result of clustering that is performed in the cloud environment 2 and that is obtained by the acquisition unit 110. The arithmetic processing performed by the arithmetic unit 150 will be described in detail later.

Thus, the functional configuration example of the information processing apparatus 100 according to one embodiment of the present disclosure has been described above. Next, an operation example of the information processing system according to one embodiment of the present disclosure will be described.

1.3. Operation Example

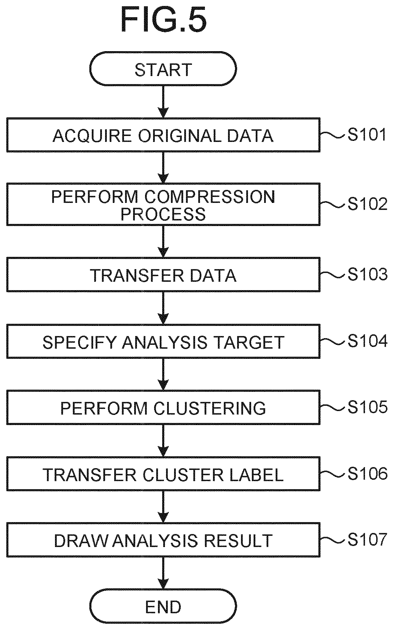

First, a flow of analyzing the measurement result obtained by the flow cytometer 10 in the cloud environment 2, and drawing an analysis result in the local environment 1 will be described. FIG. 5 is a flow diagram illustrating an operation example of the information processing system according to one embodiment of the present disclosure. FIG. 5 illustrates a flow of analyzing the measurement result obtained by the flow cytometer 10 in the cloud environment 2, and drawing an analysis result in the local environment 1. The operation example of the information processing system according to one embodiment of the present disclosure will be described below with reference to FIG. 5.

First, in the local environment 1, the information processing apparatus 100 acquires original data (measurement result) that is a basis of analysis from the flow cytometer 10 (Step S101). Upon acquiring the original data from the flow cytometer 10, the information processing apparatus 100 performs the compression process on the original data (Step S102). This compression process is performed by, for example, the compression unit 120. Then, the information processing apparatus 100 transfers the data subjected to the compression process to the cloud environment 2 (Step S103). This transfer process is performed by, for example, the transmission unit 130. By performing the compression process on the original data, it is possible to reduce the amount of data to be transferred from the local environment 1 to the cloud environment 2, and it is possible to reduce a transfer time of the data to be transferred from the local environment 1 to the cloud environment 2.

Subsequently, the information processing apparatus 100 specifies an analysis target in the cloud environment 2 (Step S104). The analysis target may be specified by the user of the information processing apparatus 100. Meanwhile, this step may be omitted if all pieces of data are adopted as analysis targets in the cloud environment 2.

Subsequently, in the cloud environment 2, the clustering processing apparatus 20 performs the clustering process on the measurement result received from the information processing apparatus 100 (Step S105). If the analysis target is specified, the clustering processing apparatus 20 performs the clustering process on the specified target.

After performing the clustering process, the clustering processing apparatus 20 transfers a cluster label to the local environment 1 (Step S106). The information processing apparatus 100 performs the process of drawing the analysis result on the basis of the received cluster label (Step S107). This process of drawing the analysis result is performed by, for example, the display control unit 140.

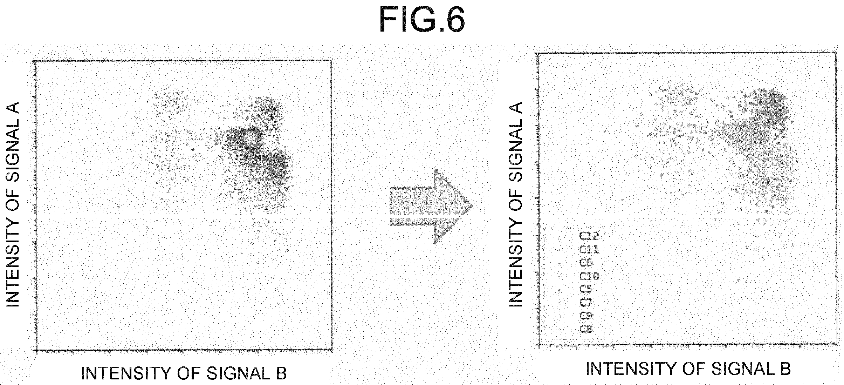

The information processing apparatus 100 according to the present embodiment, in performing histogram plot or two-dimensional plot using the original data associated with the data ID or the like and thereafter displaying a heat map or the like when drawing the analysis result, creates a visualization by performing coloring in accordance with the cluster label. FIG. 6 is an explanatory diagram illustrating an example of how the analysis result is drawn by the information processing apparatus 100. In each of the graphs illustrated in FIG. 6, a vertical axis and a horizontal axis represent intensities of two signals. Here, the two signals are referred to as a "signal A" and a "signal B". The graph on the left side in FIG. 6 is a drawing example of normal density plot. The information processing apparatus 100 may perform coloring in accordance with the cluster label to create a visualization as in the right side in FIG. 6.

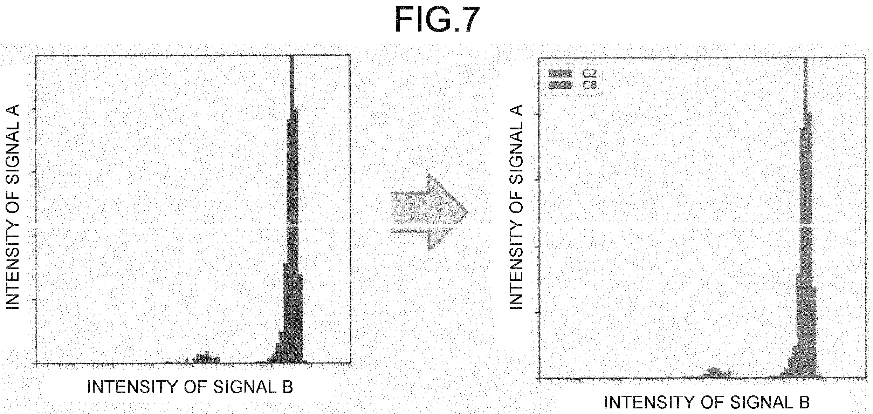

FIG. 7 is an explanatory diagram illustrating an example of how the analysis result is drawn by the information processing apparatus 100. In each of the graphs illustrated in FIG. 7, a vertical axis and a horizontal axis represent intensities of two signals. Here, the two signals are referred to as the "signal A" and the "signal B". The graph on the left side in FIG. 7 is a drawing example of normal histogram plot. The information processing apparatus 100 may perform coloring in accordance with the cluster label to create a visualization as in the right side in FIG. 7.

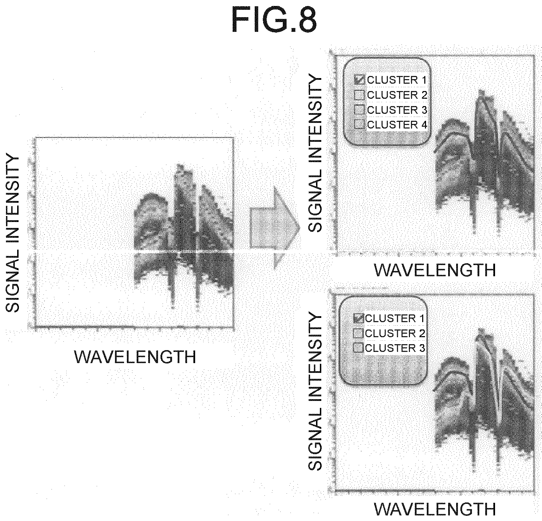

Further, the information processing apparatus 100 may overlay a representative value (for example, a median value, an average value, or the like) that is calculated for each of clusters with respect to the spectrum plot. Furthermore, for example, it may be possible to change a color or a type (dotted line, dashed line, or the like) of the representative value for each of the clusters. By overlaying the representative value calculated for each of the clusters, it is possible to allow the user to intuitively recognize expression property. FIG. 8 is an explanatory diagram illustrating an example of how the analysis result is drawn by the information processing apparatus 100. FIG. 8 illustrates an example in which the cluster result is overlaid on the spectrum plot.

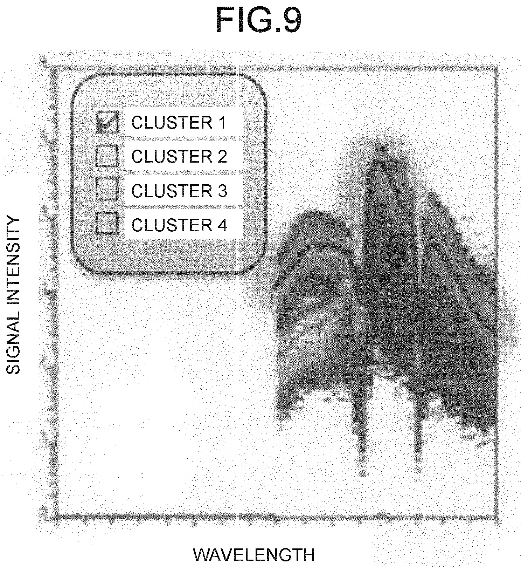

The representative value need not always be a single thin line, but may be displayed such that variation in a data distribution in the cluster can be clarified. For example, it may be possible to determine an existence range by a maximum value, a minimum value, standard deviation, and a quartile of the cluster, and indicate them by color gradation, auxiliary lines, or the like. FIG. 9 is an explanatory diagram illustrating an example of how the analysis result is drawn by the information processing apparatus 100. FIG. 9 illustrates an example in which the cluster result is overlaid on the spectrum plot.

Furthermore, even when performing the clustering again by changing the analysis target, the information processing apparatus 100 need mot transfer all pieces of data acquired from the flow cytometer 10 again, but it is sufficient to transfer only the data ID of the analysis target. The information processing apparatus 100 is able to immediately perform the drawing process again by only transferring the data ID of the analysis target.

The cloud model including the local environment 1 and the cloud environment 2 as the information processing system has been described above, but the present disclosure is not limited to this example. Caching the data subjected to the compression process in the local environment 1 has an advantage in that the compression process on large-scale data need not be performed, so that there is an advantage in a case in which, as the information processing system, operation is completed in the local environment without data transfer to the cloud environment. By caching the data subjected to the compression process in the local environment 1, it is further possible to perform drawing and clustering in parallel to each other, so that it is possible to reduce time needed to draw the analysis result.

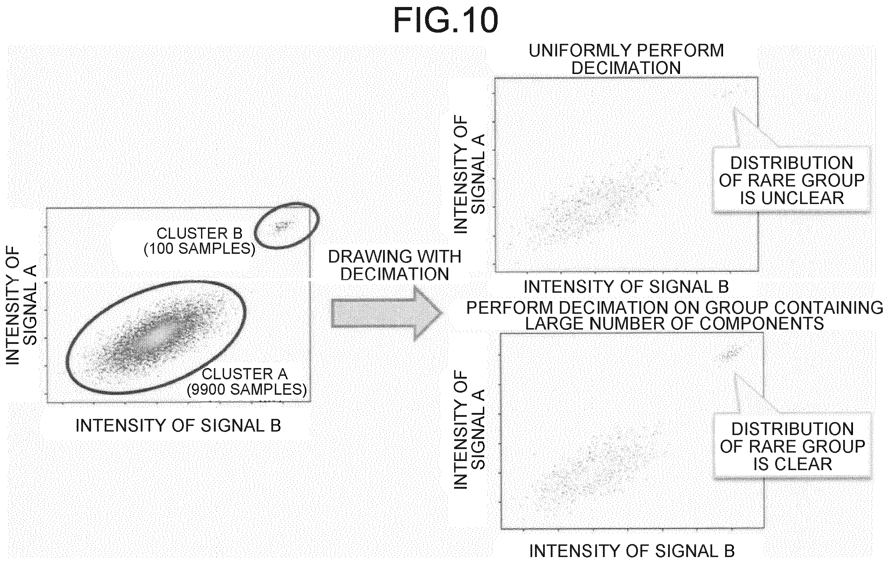

The number of pieces of data output by the flow cytometer 10 is huge. Therefore, to increase a speed of the process of drawing the analysis result, sampling may be performed at the time of displaying the two-dimensional plot or the like. To increase the speed of the process of drawing the analysis result, for example, decimation may uniformly be performed at a certain rate. However, if sampling is performed randomly, the number of rare groups present at a low rate is reduced and a distribution may become unclear. FIG. 10 is an explanatory diagram illustrating an example of a visualization by decimation using a cluster label. In each of the graphs illustrated in FIG. 10, a vertical axis and a horizontal axis represent intensities of two signals. Here, the two signals are referred to as the "signal A" and the "signal B". For example, it is assumed that a cluster A has 9900 samples, and a cluster B has 100 samples. In this state, if decimation is uniformly performed on the two clusters, a distribution of the cluster B becomes unclear.

To cope with this, the information processing apparatus 100 according to one embodiment of the present disclosure may perform sub-sampling on only a group containing a large number of components by using the clustering result, and display a distribution of a rare group in an easily understandable manner.

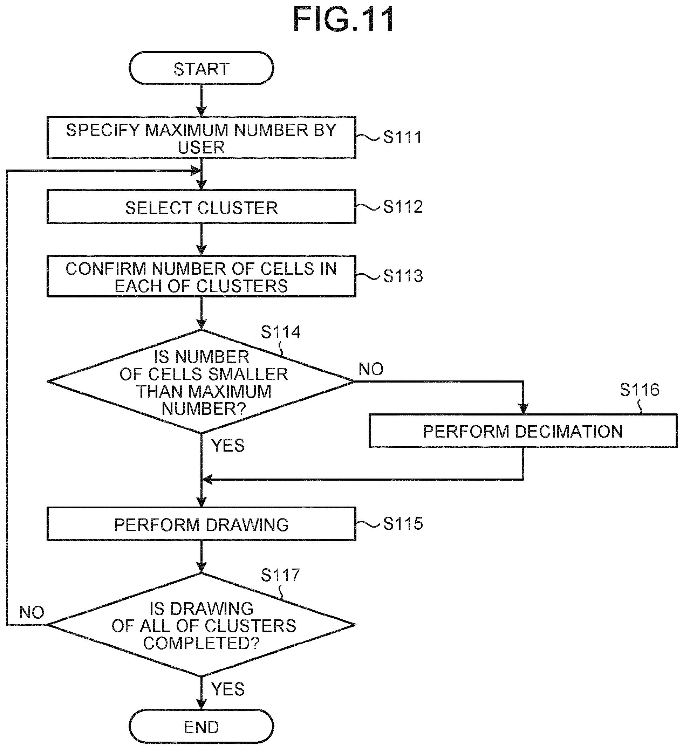

FIG. 11 is a flow diagram illustrating an operation example of the information processing apparatus 100 according to one embodiment of the present disclosure. FIG. 11 illustrates an operation example of the information processing apparatus 100 when performing sub-sampling on only a group containing a large number of components by using the clustering result. The operation example of the information processing apparatus 100 according to one embodiment of the present disclosure will be described below with reference to FIG. 11.

The user of the information processing apparatus 100 specifies the maximum number of pieces of data to be drawn (Step S111). By specifying the maximum number of pieces of data to be drawn, the user of the information processing apparatus 100 is able to arbitrarily select a balance between a drawing speed of the information processing apparatus 100 and the amount of data to be drawn by the information processing apparatus 100.

Subsequently, the information processing apparatus 100 selects a single cluster (Step S112), and confirms the number of cells for each of the clusters (Step S113). The information processing apparatus 100 determines whether the confirmed number of cells is smaller than the maximum number that is specified by the user at Step S111 as described above (Step S114). If the number of cells is smaller than the maximum number (Step S114, Yes), the information processing apparatus 100 draws the data without performing decimation (Step S115). In contrast, if the number of cells is not smaller than the maximum number (Step S114, No), the information processing apparatus 100 performs decimation on the data to be drawn (Step S116).

Thereafter, the information processing apparatus 100 determines whether drawing of all of the clusters is completed (Step S117), and if drawing of all of the clusters is not completed (Step S117, No), the information processing apparatus 100 returns to Step S112 as described above, and selects a different cluster that is not drawn. If drawing of all of the clusters is completed (Step S117, Yes), the information processing apparatus 100 terminates the series of processes. By performing the series of operation as described above, the information processing apparatus 100 according to one embodiment of the present disclosure performs sampling on only a group containing a large number of components by using the clustering result, so that it is possible to display a distribution of a rare group in an easily understandable manner.

If the user of the information processing apparatus 100 specifies all pieces of data as data to be drawn, the information processing apparatus 100 may plot the data little by little to reduce a drawing wait time. In this case, the information processing apparatus 100 performs drawing for each certain number of pieces of data for each of the clusters by using the cluster label. By causing the information processing apparatus 100 to perform drawing for each certain number of pieces of data for each of the clusters by using the cluster label, the user is able to rapidly obtain an image representing the distribution from the information processing apparatus 100.

As described above, in existing clustering processes, there may be a case that needs to be excluded from a cluster, such as a case that is largely deviated from a distribution of a group or a case that is difficult to be distinguished from other clusters because of a close distance to the other clusters. In the existing clustering processes, by manually eliminating components at ends of the group and performing gating, it is possible to exclude the above-described data. However, in the case of a multidimensional fluorescence signal that is obtained from the flow cytometer, it is necessary to perform plot by selecting a single axis or two axes and thereafter select a range, which is cumbersome for a user.

To cope with this, the information processing apparatus 100 according to one embodiment of the present disclosure provides an analysis function to calculate, for each piece of data, a degree of deviation from a cluster to which each piece of data belongs, and easily exclude deviated data.

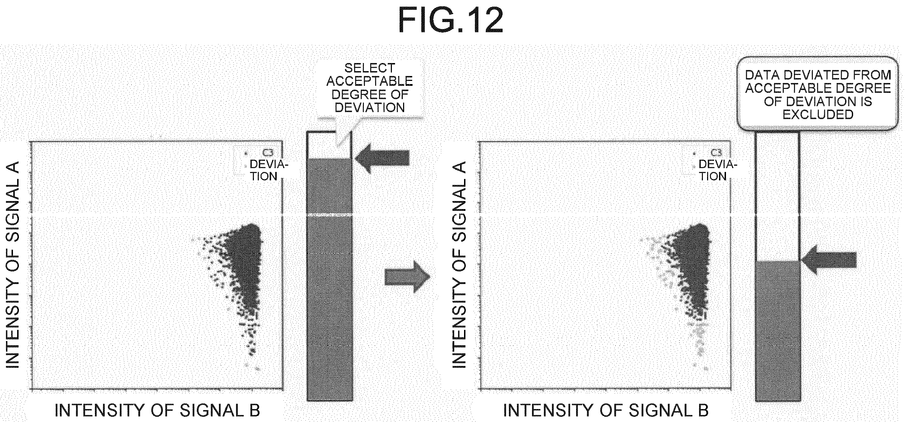

FIG. 12 is an explanatory diagram illustrating an example of a user interface provided by the information processing apparatus 100 according to one embodiment of the present disclosure. In each of graphs that are drawn by the user interface illustrated in FIG. 12, a vertical axis and a horizontal axis represent intensities of two signals. Here, the two signals are referred to as the "signal A" and the "signal B". The information processing apparatus 100 prepares a user interface that allows a user to select an acceptable degree of deviation as illustrated in FIG. 12. If the user selects the acceptable degree of deviation, the information processing apparatus 100 excludes data deviated from the acceptable degree of deviation from the group and thereafter perform drawing, in accordance with the selection of the user. In this manner, by arranging a simple user interface, the information processing apparatus 100 according to one embodiment of the present disclosure is able to easily exclude deviated data by only requesting the user to indicate a single index.

When performing drawing while excluding the data deviated from the acceptable degree of deviation from the group, the information processing apparatus 100 may display the deviated data in a different color from non-deviated data, or may change transparency (transmissivity) in display. When performing drawing while excluding the data deviated from the acceptable degree of deviation from the group, the information processing apparatus 100 may perform what is called preview display in accordance with operation performed on the user interface by the user.

Further, the information processing apparatus 100 is able to allow the user to intuitively recognize which data can be excluded when the index is changed, by using the spectrum plot, a T-SNE graph, or the like in addition to the arbitrary two-dimensional plot as illustrated in FIG. 12. The user confirms the acceptable degree of deviation by viewing the interface. The information processing apparatus 100 performs drawing while excluding unclear data from each of the clusters on the basis of the confirmed degree of deviation.

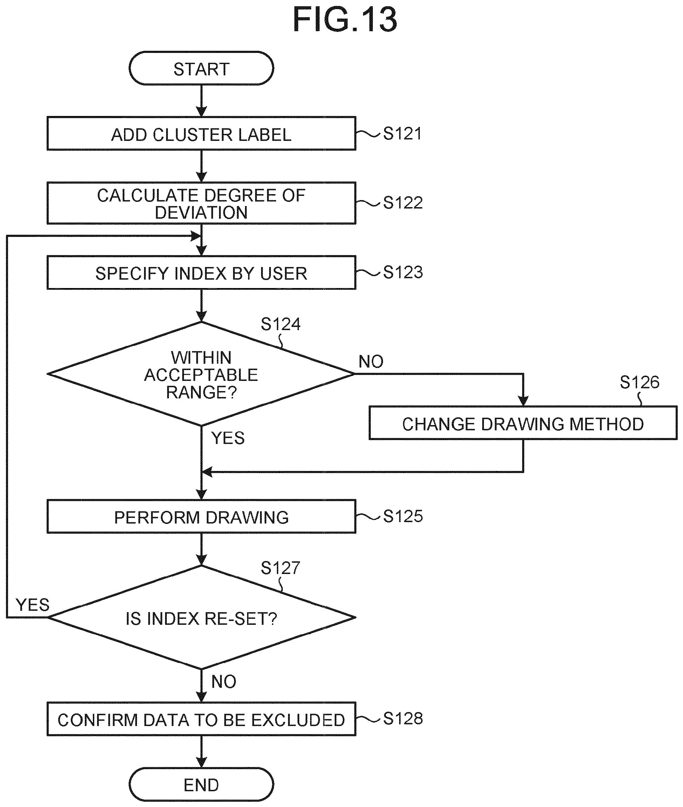

FIG. 13 is a flow diagram illustrating an operation example of the information processing apparatus 100 according to one embodiment of the present disclosure. The operation example of the information processing apparatus 100 according to one embodiment of the present disclosure will be described below with reference to FIG. 13.

First, labeling is performed by using any of methods on each piece of cell data obtained from the flow cytometer 10 (Step S121). This process may be performed by the clustering as described above, or by using conventional manual gating.

Subsequently, the information processing apparatus 100 calculates a degree of deviation from the cluster to which each piece of data belongs, with respect to each piece of data (Step S122). This calculation is performed by, for example, the arithmetic unit 150. A method of calculating the degree of deviation is not specifically limited. Examples of the method of calculating the degree of deviation will be described below.

1. Index based on distance from belonging group

The information processing apparatus 100 may calculate the degree of deviation by using a distance from a belonging group. For example, the information processing apparatus 100 may calculate the degree of deviation on the basis of a distance from a barycenter of the belonging group, or may calculate the degree of deviation on the basis of an index in which a density of a distribution is taken into account as indicated by Expression (1) below.

$$A(i) = \frac{r(i)}{r(i')} \tag{1}$$

In Expression (1) above, $r(i)$ is a distance from a target sample $X(i)$ to a nearest sample $X(i')$, and $r(i')$ is a distance between $X(i')$ and a nearest sample.

2. Index calculated based on distance from belonging group and distance from non-belonging group

The information processing apparatus 100 may calculate the degree of deviation on the basis of a distance from a belonging group and a distance from a non-belonging group. In this case, the information processing apparatus 100 additionally use the distance from non-belonging group in order to take into account boundaries among a plurality of clusters. As one example, a silhouette coefficient may be adopted. The silhouette coefficient for the sample $X(i)$ is defined by Expression (2) below.

$$s(i) = \frac{b(i) - a(i)}{max\{a(i), b(i)\}} \tag{2}$$

In Expression (2) above, $a(i)$ is an average distance from all other samples in the same cluster as the sample $X(i)$, and indicates an agglomeration degree with respect to a belonging cluster. Further, in Expression (2) above, $b(i)$ is an average distance from all of samples in a cluster located closest to the sample $X(i)$, and indicates a degree of deviation from the non-belonging neighboring cluster.

3. Index calculated by cluster ensemble

The indices described in the items 1. and 2. above are indices based on distances. If a cluster ensemble method for obtaining a final clustering result by using results obtained by a plurality of number of times of clustering is used at the time of labeling, it is possible to calculate reliability for each piece of data. In this case, the information processing apparatus 100 adopts data with low reliability as the degree of deviation.

In the clustering, data is basically separated into a plurality of clusters on the basis of a specific rule, so that it is not always possible to obtain a separation result as desired by the user. Therefore, the user sometimes performs additional analysis to determine whether the generated cluster is correct, but it is cumbersome for the user to perform the additional analysis on a large number of clusters. Here, a function capable of reducing time and effort of the user for the additional analysis will be proposed.

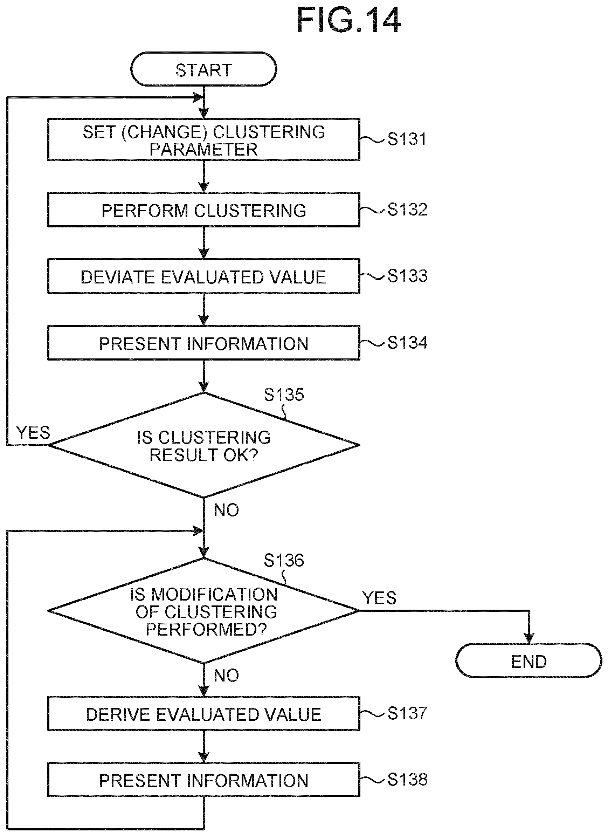

FIG. 14 is a flow diagram illustrating an operation example of the information processing apparatus 100 according to one embodiment of the present disclosure. FIG. 14 illustrates an operation example of the information processing apparatus 100 for reducing time and effort of the user for the additional analysis. The operation example of the information processing apparatus 100 according to one embodiment of the present disclosure will be described below with reference to FIG. 14.

The user sets a clustering parameter (Step S131). Subsequently, the clustering processing apparatus 20 performs clustering by using any of methods on the basis of the set parameter (Step S132). If the parameter used for the clustering is present at the time of the clustering, the clustering processing apparatus 20 records the parameter. Examples of the parameter include one, such as the number of clusters or the number of dimensions to be used, which is used independent of the clustering method, and a parameter unique to the cluster method.

Thereafter, the information processing apparatus 100 derives an evaluated value (reliability) of the cluster, on the basis of the calculated cluster (Step S133). This derivation process is performed by, for example, the arithmetic unit 150. Examples of the cluster evaluated value may include those as described below.

The cluster evaluated value may include an evaluated value obtained by the cluster ensemble. As described above, the cluster ensemble method for extracting a single reliable clustering result from a plurality of number of times of clustering includes a method, such as Meta-Clustering Algorithm (MCLA) or a Co-association Matrix method, capable of calculating reliability of all of the clusters or a method of calculating reliability of each of the clusters. With use of the methods as described above, in many cases, it is possible to calculate the degree of reliability with which each piece of data belongs to a finally belonging cluster as described above. Therefore, it is possible to calculate an evaluated value of the entire clustering result by calculating an average of all pieces of data, and it is possible to calculate an evaluated value of each of the clusters by calculating an average for each of the clusters.

The cluster evaluated value may include Clustering Validity Indices (CVI). The CVI is a cluster evaluated value that is calculated based on a degree of separation from other clusters or a density of a distribution of data in the cluster, on the basis of data distributions inside and outside of the cluster. As one example, the silhouette coefficient indicated by Expression above, a Davies Bouldin index (DBI), a COP index, and the like may be adopted. In the case of the silhouette coefficient, a calculation using the above-described Expression is performed for each piece of data, so that it is possible to calculate an evaluated value of the entire clustering result by calculating an average of all pieces of data, and it is possible to calculate an evaluated value of each of the clusters by calculating an average for each of the clusters.

Subsequently, the information processing apparatus 100 presents the calculated evaluated value of the entire clustering result (Step S134). The information processing apparatus 100 displays the evaluated value of the entire clustering result, so that if the evaluated value is extremely negative (Step S135, No), the user is able to change the parameter of the clustering before performing the additional analysis of the cluster, and give an instruction to perform the clustering again. Further, if the clustering is performed again, by memorizing a previous clustering result and a previous evaluated value, it is possible to evaluate whether the re-performed clustering result is improved.

Thereafter, if the clustering evaluated value is as desired by the user (Step S135, Yes), the user determines whether to perform additional modification of the cluster with respect to a certain clustering result (Step S136). If the user determines that the additional modification of the cluster is performed, the information processing apparatus 100 derives an evaluated value again (Step S137), and presents information on the derived evaluated value (Step S138). In this case, the evaluated value of each of the clusters may be used. The information processing apparatus 100 may use the evaluated value to highlight a cluster with a low evaluated value for which the additional analysis needs to be performed, or a cluster with a high evaluated value for which the additional analysis need not be performed, instead of displaying the evaluated value as it is with respect to each of the clusters. In any case, the user need not analyze all of the clusters by using the display information.

Furthermore, if integration or separation of clusters is performed due to the additional analysis performed by the user, the information processing apparatus 100 recalculates a cluster evaluated value, and presents evaluated values obtained before and after modification of the cluster. By presenting the evaluated values obtained before and after modification of the cluster, the information processing apparatus 100 gives a feedback of an evaluation result with respect to manual modification performed by the user. Consequently, the user is able to use an objective evaluation index and modify the cluster.

In the explanation above, the information processing apparatus 100 performs the compression process on the original data upon acquiring the original data from the flow cytometer 10, but the present disclosure is not limited to this example. The information processing apparatus 100 may extract spectrum data from the measurement result obtained by the flow cytometer 10, and perform operation of transferring the extracted data to the cloud environment 2.

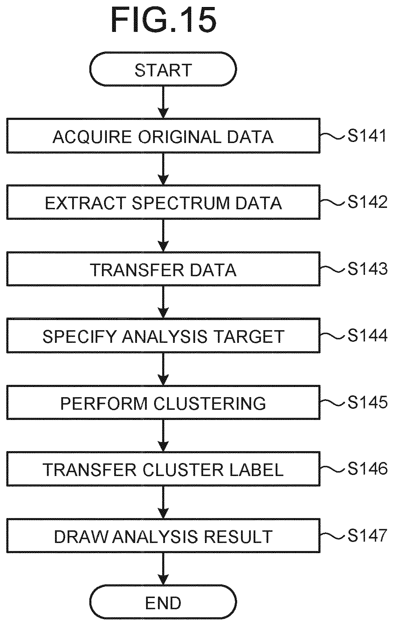

FIG. 15 is a flow diagram illustrating an operation example of the information processing system according to one embodiment of the present disclosure. FIG. 15 illustrates a flow of analyzing the measurement result obtained by the flow cytometer 10 in the cloud environment 2, and drawing an analysis result in the local environment 1. The operation example of the information processing system according to one embodiment of the present disclosure will be described below with reference to FIG. 5.

First, in the local environment 1, the information processing apparatus 100 acquires original data (measurement data) that is a basis of analysis from the flow cytometer 10 (Step S141). Upon acquiring the original data from the flow cytometer 10, the information processing apparatus 100 performs the process of extracting spectrum data from the original data (Step S142). Then, the information processing apparatus 100 transfers the extracted data to the cloud environment 2 (Step S143). This transfer process is performed by, for example, the transmission unit 130. By performing the compression process on the original data, it is possible to reduce the amount of data to be transferred from the local environment 1 to the cloud environment 2, and it is possible to reduce a transfer time of the data to be transferred from the local environment 1 to the cloud environment 2.

Subsequently, the information processing apparatus 100 specifies an analysis target in the cloud environment 2 (Step S144). The analysis target is specified by the user of the information processing apparatus 100. Meanwhile, this step may be omitted if all pieces of data are adopted as analysis targets in the cloud environment 2.

Subsequently, in the cloud environment 2, the clustering processing apparatus 20 performs the clustering process on the measurement result received from the information processing apparatus 100 (Step S145). If the analysis target is specified, the clustering processing apparatus 20 performs the clustering process on the specified target.

After performing the clustering process, the clustering processing apparatus 20 transfers a cluster label to the local environment 1 (Step S146). The information processing apparatus 100 performs the process of drawing the analysis result on the basis of the received cluster label (Step S147). This process of drawing the analysis result is performed by, for example, the display control unit 140.

Each of the functions described in the present embodiment may be used for clustering for which data of a conventional flow cytometer is used as an input instead of using spectral data as an input.

2. Hardware Configuration

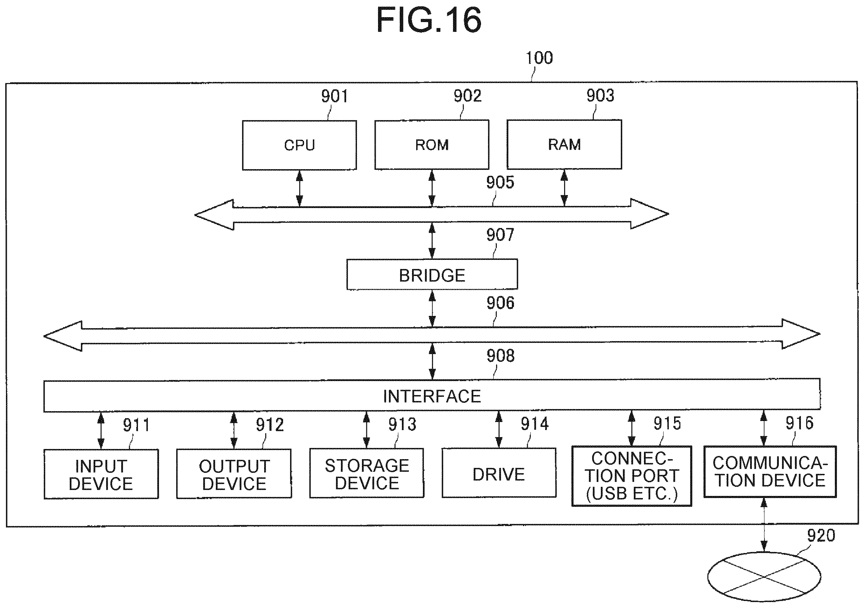

A hardware configuration of the information processing apparatus 100 according to the present embodiment will be described below with reference to FIG. 16. FIG. 16 is a block diagram illustrating one example of the hardware configuration of the information processing apparatus 100 according to the present embodiment.

As illustrated in FIG. 16, the information processing apparatus 100 includes a central processing unit (CPU) 901, a read only memory (ROM) 902, a random access memory (RAM) 903, a bridge 907, internal buses 905 and 906, an interface 908, an input device 911, an output device 912, a storage device 913, a drive 914, a connection port 915, and a communication device 916.

The CPU 901 functions as an arithmetic processing device and a control device, and controls the entire operation of the information processing apparatus 100 in accordance with various programs stored in the ROM 902 or the like. The ROM 902 stores therein programs and arithmetic parameters used by the CPU 901, and the RAM 903 temporarily stores therein programs used during execution of the CPU 901, parameters that are appropriately changed during the execution, and the like. For example, the CPU 901 may implement the functions of the compression unit 120 and the display control unit 140.

The CPU 901, the ROM 902, and the RAM 903 are connected to each other by the bridge 907, the internal buses 905 and 906, and the like. Further, the CPU 901, the ROM 902, and the RAM 903 are also connected to the input device 911, the output device 912, the storage device 913, the drive 914, the connection port 915, and the communication device 916 via the interface 908.

The input device 911 includes an input device, such as a touch panel, a keyboard, a mouse, a button, a microphone, a switch, and a lever, by which information is input. Further, the input device 911 includes an input control circuit for generating an input signal based on the input information and outputting the input signal to the CPU 901.

The output device 912 includes, for example, a display device, such as a cathode ray tube (CRT) display device, a liquid crystal display device, and an organic electroluminescence (EL) display device. Further, the output device 912 may include a voice output device, such as a speaker and a headphone. For example, the output device 912 may implement the function of the display control unit 140.

The storage device 913 is a storage device for storing data of the information processing apparatus 100. The storage device 913 may include a storage medium, a storage device that stores data in the storage medium, a reading device that reads data from the storage medium, and a deleting device that deletes stored data.

The drive 914 is a reader-writer for a storage medium, and is incorporated in or externally attached to the information processing apparatus 100. For example, the drive 914 reads information that is stored in an attached removable storage medium, such as a magnetic disk, an optical disk, or a semiconductor memory, and outputs the information to the RAM 903. The drive 914 is able to write information to the removable storage medium.

The connection port 915 is, for example, a connection interface including a connection port, such as a universal serial bus (USB) port, an Ethernet (registered trademark) port, a port compliant with IEEE802.11, or an optical audio terminal, for connecting an external connection device.

The communication device 916 is, for example, a communication interface configured with a communication device or the like for establishing a connection to a network 920. Further, the communication device 916 may be a communication device compatible with a wired LAN or a wireless LAN, or a cable communication device that performs cable communication in a wired manner. The communication device 916 and the connection port 915 may implement the functions of the acquisition unit 110 and the transmission unit 130.

Meanwhile, it is possible to generate a computer program for implementing the same functions as those of each of the components of the information processing apparatus according to the present embodiment as described above, with respect to hardware, such as the CPU, the ROM, and the RAM, included in the information processing apparatus 100. Furthermore, it is possible to provide a storage medium that stores therein the computer program.

3. Conclusion

As described above, according to one embodiment of the present disclosure, it is possible to provide the information processing apparatus 100 that is able to reduce a processing time at the time of performing clustering on information obtained from a flow cytometer, and reduce a drawing time of a clustering result.

Each of Steps in processes performed by each of the apparatuses in the present specification need not always be chronologically performed in the same order as illustrated in the sequence diagrams or the flowcharts. For example, each of Steps in the processes performed by each of the apparatuses may be performed in different order from the order illustrated in the sequence diagrams and the flowcharts, or may be performed in a parallel manner.

While the preferred embodiments of the present disclosure have been described in detail above with reference to the accompanying drawings, the technical scope of the present disclosure is not limited to the examples as described above. It is obvious that a person skilled in the technical field of the present disclosure may conceive various alternations and modifications within the scope of the appended claims, and it should be understood that they will naturally come under the technical scope of the present disclosure.

Further, the effects described in this specification are merely illustrative or exemplified effects, and are not limitative. That is, with or in the place of the above effects, the technology according to the present disclosure may achieve other effects that are clear to those skilled in the art from the description of this specification.

Reference Signs List

| | |
|---|---|
| 1 | local environment |
| 2 | cloud environment |
| 3 | network |
| 10 | flow cytometer |
| 11 | laser light source |
| 13 | flow cell |
| 15 | optical detector |
| 20 | clustering processing apparatus |
| 100 | information processing apparatus |
| 140 | display control unit |
| 151 | light-receiving element array |
| 153 | detector |

**Claims**

1. An information processing apparatus comprising:

   a compression unit (120) that performs a compression process on a data amount of spectral data that is measured when a particle is irradiated with a laser beam to obtain compressed spectral data having a one-to-one correspondence to the measured spectral data;
   an acquisition unit (110) that acquires a result of a clustering process performed on the compressed spectral data; and
   an output unit that outputs an analysis result using the measured spectral data and the result of the clustering process acquired by the acquisition unit.

2. The information processing apparatus according to claim 1, wherein the output unit outputs the result of the clustering process with respect to a specified target.

3. The information processing apparatus according to claim 1 or claim 2, wherein the output unit outputs a predetermined value that is calculated for each of clusters, in a superimposed manner on the result of the clustering process.

4. The information processing apparatus according to any of claims 1 to 3, wherein the output unit performs sampling on the result of the clustering process, and thereafter outputs the result of the clustering process.

5. The information processing apparatus according to claim 4, wherein the output unit changes contents of the sampling on the basis of a setting on a specified threshold, and thereafter outputs the result of the clustering process.

6. The information processing apparatus according to any of claims 1 to 5, wherein the output unit calculates a degree of deviation from a cluster with respect to the result of the clustering process.

7. The information processing apparatus according to claim 6, wherein the output unit outputs data for which the degree of deviation is equal to or larger than a specified threshold, in a different manner from data smaller than the threshold.

8. The information processing apparatus according to claim 7, wherein the output unit outputs the data for which the degree of deviation is equal to or larger than the specified threshold, in a different color or with different transparency from the data smaller than the threshold.

9. The information processing apparatus according to claim 7, wherein the output unit dynamically changes display of the data equal to or larger than the threshold and the data smaller than the threshold, in accordance with a dynamical change of the threshold.

10. The information processing apparatus according to any of claims 1 to 9, wherein the output unit outputs data compressed by the compression unit to a different device that is connected via a network.

11. The information processing apparatus according to claim 10, wherein the result of the clustering process is acquired from the different device.

12. The information processing apparatus according to any of claims 1 to 11, wherein the spectral data is data output from a flow cytometer (10).

13. The information processing apparatus according to any of claims 1 to 12, wherein the output unit controls display of the result of the clustering process.

14. An information processing method implemented by a processor, the information processing method comprising:

performing a compression process on a data amount of spectral data that is measured when a particle is irradiated with a laser beam to obtain compressed spectral data having a one-to-one correspondence to the measured spectral data;
acquiring a result of a clustering process performed on the compressed spectral data; and
outputting an analysis result using the measured spectral data and the acquired result of the clustering process.

15. A computer program that causes a computer to execute the information processing method according to claim 14.

**Patentansprüche**

1. Informationsverarbeitungseinrichtung, umfassend:

eine Komprimierungseinheit (120), die einen Komprimierungsprozess an einer Datenmenge von Spektraldaten durchführt, die gemessen werden, wenn ein Partikel mit einem Laserstrahl bestrahlt wird, um komprimierte Spektraldaten zu erhalten, die eine Eins-zu-Eins-Entsprechung zu den gemessenen Spektraldaten aufweisen; eine Erfassungseinheit (110), die ein Ergebnis eines Clustering-Prozesses erfasst, der an den komprimierten Spektraldaten durchgeführt wurde; und eine Ausgabeeinheit, die ein Analyseergebnis unter Verwendung der gemessenen Spektraldaten und des Ergebnisses des Clustering-Prozesses ausgibt, das von der Erfassungseinheit erfasst wurde.

2. Informationsverarbeitungseinrichtung nach Anspruch 1, wobei die Ausgabeeinheit das Ergebnis des Clustering-Prozesses in Bezug auf ein angegebenes Ziel ausgibt.

3. Informationsverarbeitungseinrichtung nach Anspruch 1 oder Anspruch 2, wobei die Ausgabeeinheit einen vorgegebenen Wert, der für jeden der Cluster berechnet wird, in einer dem Ergebnis des Clustering-Prozesses überlagerten Weise ausgibt.

4. Informationsverarbeitungseinrichtung nach einem der Ansprüche 1 bis 3, wobei die Ausgabeeinheit ein Abtasten des Ergebnisses des Clustering-Prozesses durchführt und danach das Ergebnis des Clustering-Prozesses ausgibt.

**5.** Informationsverarbeitungseinrichtung nach Anspruch 4, wobei die Ausgabeeinheit Inhalte des Abtastens auf der Grundlage einer Einstellung an einem angegebenen Schwellenwert ändert und danach das Ergebnis des Clustering-Prozesses ausgibt.

**6.** Informationsverarbeitungseinrichtung nach einem der Ansprüche 1 bis 5, wobei die Ausgabeeinheit einen Grad der Abweichung von einem Cluster in Bezug auf das Ergebnis des Clustering-Prozesses berechnet.

**7.** Informationsverarbeitungseinrichtung nach Anspruch 6, wobei die Ausgabeeinheit Daten, für die der Grad der Abweichung gleich oder größer als ein angegebener Schwellenwert ist, in einer anderen Weise ausgibt als Daten, die kleiner als der Schwellenwert sind.

**8.** Informationsverarbeitungseinrichtung nach Anspruch 7, wobei die Ausgabeeinheit die Daten, für die der Grad der Abweichung gleich oder größer als der angegebene Schwellenwert ist, in einer anderen Farbe oder mit anderer Transparenz ausgibt als die Daten, die kleiner als der Schwellenwert sind.

**9.** Informationsverarbeitungseinrichtung nach Anspruch 7, wobei die Ausgabeeinheit die Anzeige der Daten, die gleich oder größer als der Schwellenwert sind, und der Daten, die kleiner als der Schwellenwert sind, gemäß einer dynamischen Änderung des Schwellenwerts dynamisch ändert.

**10.** Informationsverarbeitungseinrichtung nach einem der Ansprüche 1 bis 9, wobei die Ausgabeeinheit Daten, die durch die Komprimierungseinheit komprimiert wurden, an eine andere Vorrichtung ausgibt, die über ein Netzwerk angeschlossen ist.

**11.** Informationsverarbeitungseinrichtung nach Anspruch 10, wobei das Ergebnis des Clustering-Prozesses von der anderen Vorrichtung erfasst wird.

**12.** Informationsverarbeitungseinrichtung nach einem der Ansprüche 1 bis 11, wobei es sich bei den Spektraldaten um Daten handelt, die aus einem Durchflusszytometer (10) ausgegeben werden.

**13.** Informationsverarbeitungseinrichtung nach einem der Ansprüche 1 bis 12, wobei die Ausgabeeinheit die Anzeige des Ergebnisses des Clustering-Prozesses steuert.

**14.** Informationsverarbeitungsverfahren, das durch einen Prozessor implementiert wird, wobei das Informationsverarbeitungsverfahren umfasst:

Durchführen eines Komprimierungsprozesses an einer Datenmenge von Spektraldaten, die gemessen werden, wenn ein Partikel mit einem Laserstrahl bestrahlt wird, um komprimierte Spektraldaten zu erhalten, die eine Eins-zu-Eins-Entsprechung zu den gemessenen Spektraldaten aufweisen;
Erfassen eines Ergebnisses eines Clustering-Prozesses, der an den komprimierten Spektraldaten durchgeführt wurde; und
Ausgeben eines Analyseergebnisses unter Verwendung der gemessenen Spektraldaten und des erfassten Ergebnisses des Clustering-Prozesses.

**15.** Computerprogramm, das einen Computer veranlasst, das Informationsverarbeitungsverfahren nach Anspruch 14 auszuführen.

**Revendications**

**1.** Appareil de traitement d'informations comprenant :

une unité de compression (120) qui effectue un processus de compression sur une quantité de données de données spectrales mesurées lorsqu'une particule est irradiée par un faisceau laser afin d'obtenir des données spectrales comprimées ayant une correspondance biunivoque avec les données spectrales mesurées ;
une unité d'acquisition (110) qui acquiert le résultat d'un processus de regroupement effectué sur les données spectrales comprimées ; et
une unité de sortie qui produit en sortie un résultat d'analyse en utilisant les données spectrales mesurées et le résultat du processus de regroupement acquis par l'unité d'acquisition.

2.  Appareil de traitement d'informations selon la revendication 1, dans lequel l'unité de sortie produit en sortie le résultat du processus de regroupement par rapport à une cible spécifiée.

3.  Appareil de traitement d'informations selon la revendication 1 ou la revendication 2, dans lequel l'unité de sortie produit en sortie une valeur prédéterminée calculée pour chacun des regroupements, de manière superposée au résultat du processus de regroupement.

4.  Appareil de traitement d'informations selon l'une quelconque des revendications 1 à 3, dans lequel l'unité de sortie effectue un échantillonnage sur le résultat du processus de regroupement, puis produit en sortie le résultat du processus de regroupement.

5.  Appareil de traitement d'informations selon la revendication 4, dans lequel l'unité de sortie modifie le contenu de l'échantillonnage sur la base d'un réglage d'un seuil spécifié, et produit ensuite en sortie le résultat du processus de regroupement.

6.  Appareil de traitement d'informations selon l'une quelconque des revendications 1 à 5, dans lequel l'unité de sortie calcule un degré de déviation d'un regroupement par rapport au résultat du processus de regroupement.

7.  Appareil de traitement d'informations selon la revendication 6, dans lequel l'unité de sortie produit en sortie des données pour lesquelles le degré de déviation est égal ou supérieur à un seuil spécifié, d'une manière différente des données plus petites que le seuil.

8.  Appareil de traitement d'informations selon la revendication 7, dans lequel l'unité de sortie produit en sortie les données pour lesquelles le degré de déviation est égal ou supérieur au seuil spécifié, dans une couleur ou avec une transparence différente des données inférieures au seuil.

9.  Appareil de traitement d'informations selon la revendication 7, dans lequel l'unité de sortie modifie dynamiquement l'affichage des données égales ou supérieures au seuil et des données inférieures au seuil, en fonction d'un changement dynamique du seuil.

10. Appareil de traitement d'informations selon l'une quelconque des revendications 1 à 9, dans lequel l'unité de sortie produit en sortie les données compressées par l'unité de compression à un dispositif différent connecté par l'intermédiaire d'un réseau.

11. Appareil de traitement d'informations selon la revendication 10, dans lequel le résultat du processus de regroupement est acquis à partir de l'autre dispositif.

12. Appareil de traitement d'informations selon l'une quelconque des revendications 1 à 11, dans lequel les données spectrales sont des données de sortie provenant d'un cytomètre de flux (10).

13. Appareil de traitement d'informations selon l'une quelconque des revendications 1 à 12, dans lequel l'unité de sortie commande l'affichage du résultat du processus de regroupement.

14. Procédé de traitement d'informations mis en œuvre par un processeur, le procédé de traitement d'informations comprenant :

    la réalisation d'un processus de compression sur une quantité de données de données spectrales mesurées lorsqu'une particule est irradiée par un faisceau laser afin d'obtenir des données spectrales comprimées ayant une correspondance biunivoque avec les données spectrales mesurées ;
    l'acquisition du résultat d'un processus de regroupement effectué sur les données spectrales comprimées ; et
    la production d'un résultat d'analyse en utilisant les données spectrales mesurées et le résultat acquis du processus de regroupement.

15. Programme informatique destiné à amener un ordinateur à exécuter le procédé de traitement d'informations selon la revendication 14.

# FIG.1

EP 3 882 603 B1

# FIG.2

```
                                    ⊘ — S
                                    ⊘
                                    ⊘ — 13
                                    ⊘
   11                               ⊘                    15
 ┌──────────────┐                   ⊘          ┌──────────────┐
 │ LASER LIGHT  │─────────→         ⊘──────→   │   OPTICAL    │
 │   SOURCE     │                   ⊘          │  DETECTOR    │
 └──────────────┘                   ⊘          └──────────────┘
                                    │
                                    ↓
```

# FIG.3

```
              ┌──────────────┐
              │ LASER LIGHT  │ — 11
              │   SOURCE     │
              └──────────────┘
                     │
     13              │         L_F            151
      \            ┌───┐              ┌──────────────┐
   S ── ⊘ ─────────→      ─────────→  │              │
              └───┘              └──────────────┘
                     │                        │
                   L_S                        │
                     │                        │
                     ↓                        │
                  ┌─────┐                      │
                  │     │ — 153                │
                  │     │                      │
                  └─────┘                      │
                     │                    100  │
                     │          ┌──────────────┴─┐
                     │          │  INFORMATION   │
                     └─────────→│  PROCESSING    │
                                │  APPARATUS     │
                                └────────────────┘
```

# FIG.4

100

```
┌─────────────────────────────────┐
│                                 │
│   ┌─────────────────┐           │
│   │   ACQUISITION   │──110      │
│   │      UNIT       │           │
│   └─────────────────┘           │
│                                 │
│   ┌─────────────────┐           │
│   │   COMPRESSION   │──120      │
│   │      UNIT       │           │
│   └─────────────────┘           │
│                                 │
│   ┌─────────────────┐           │
│   │  TRANSMISSION   │──130      │
│   │      UNIT       │           │
│   └─────────────────┘           │
│                                 │
│   ┌─────────────────┐           │
│   │    DISPLAY      │──140      │
│   │  CONTROL UNIT   │           │
│   └─────────────────┘           │
│                                 │
│   ┌─────────────────┐           │
│   │   ARITHMETIC    │──150      │
│   │      UNIT       │           │
│   └─────────────────┘           │
│                                 │
└─────────────────────────────────┘
```

# FIG.5

START

ACQUIRE ORIGINAL DATA — S101

PERFORM COMPRESSION
PROCESS — S102

TRANSFER DATA — S103

SPECIFY ANALYSIS TARGET — S104

PERFORM CLUSTERING — S105

TRANSFER CLUSTER LABEL — S106

DRAW ANALYSIS RESULT — S107

END

# FIG.6

FIG.7

FIG.8

# FIG.9

# FIG.10

UNIFORMLY PERFORM
DECIMATION

INTENSITY OF
SIGNAL A

DISTRIBUTION
OF RARE GROUP
IS UNCLEAR

INTENSITY OF SIGNAL B

CLUSTER B
(100 SAMPLES)

INTENSITY OF
SIGNAL A

DRAWING WITH
DECIMATION

PERFORM DECIMATION ON GROUP CONTAINING
LARGE NUMBER OF COMPONENTS

CLUSTER A
(9900 SAMPLES)

INTENSITY OF SIGNAL B

INTENSITY OF
SIGNAL A

DISTRIBUTION
OF RARE GROUP
IS CLEAR

INTENSITY OF SIGNAL B

# FIG.11

START

SPECIFY MAXIMUM NUMBER BY USER — S111

SELECT CLUSTER — S112

CONFIRM NUMBER OF CELLS IN EACH OF CLUSTERS — S113

S114

IS NUMBER OF CELLS SMALLER THAN MAXIMUM NUMBER?

NO

S116

PERFORM DECIMATION

YES

PERFORM DRAWING — S115

S117

IS DRAWING OF ALL OF CLUSTERS COMPLETED?

NO

YES

END

# FIG.12

# FIG.13

START

ADD CLUSTER LABEL — S121

CALCULATE DEGREE OF DEVIATION — S122

SPECIFY INDEX BY USER — S123

S124

WITHIN ACCEPTABLE RANGE? — NO

S126

CHANGE DRAWING METHOD

YES

PERFORM DRAWING — S125

S127

IS INDEX RE-SET? — YES

NO

CONFIRM DATA TO BE EXCLUDED — S128

END

# FIG.14

START

SET (CHANGE) CLUSTERING PARAMETER ～S131

PERFORM CLUSTERING ～S132

DEVIATE EVALUATED VALUE ～S133

PRESENT INFORMATION ～S134

S135

IS CLUSTERING RESULT OK?

YES

NO

S136

IS MODIFICATION OF CLUSTERING PERFORMED?

YES

END

NO

DERIVE EVALUATED VALUE ～S137

PRESENT INFORMATION ～S138

# FIG.15

```
           ┌─────────────┐
           │    START    │
           └──────┬──────┘
                  │
                  ▼
  ┌──────────────────────────────┐
  │   ACQUIRE ORIGINAL DATA       │───S141
  └──────────────┬───────────────┘
                 │
                 ▼
  ┌──────────────────────────────┐
  │   EXTRACT SPECTRUM DATA       │───S142
  └──────────────┬───────────────┘
                 │
                 ▼
  ┌──────────────────────────────┐
  │      TRANSFER DATA            │───S143
  └──────────────┬───────────────┘
                 │
                 ▼
  ┌──────────────────────────────┐
  │   SPECIFY ANALYSIS TARGET     │───S144
  └──────────────┬───────────────┘
                 │
                 ▼
  ┌──────────────────────────────┐
  │    PERFORM CLUSTERING         │───S145
  └──────────────┬───────────────┘
                 │
                 ▼
  ┌──────────────────────────────┐
  │   TRANSFER CLUSTER LABEL      │───S146
  └──────────────┬───────────────┘
                 │
                 ▼
  ┌──────────────────────────────┐
  │    DRAW ANALYSIS RESULT       │───S147
  └──────────────┬───────────────┘
                 │
                 ▼
           ┌─────────────┐
           │     END     │
           └─────────────┘
```

# FIG.16

EP 3 882 603 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5772425 B

- WO 2017191699 A1

**Non-patent literature cited in the description**

- **DANIEL JIMENEZ-CARRETERO et al.** Flow Cytometry Data Preparation Guidelines for Improved Automated Phenotypic Analysis. *The Journal of Immunology*, 07 May 2018, vol. 200 (10), 3319-3331

- **ZARE HABIL et al.** Data Reduction for Spectral Clustering to Analyze High Throughput Flow Cytometry Data. *BMC Bioinformatics, Biomed Central*, 28 July 2010, vol. 11 (1), 403
- **KIERAN O'NEILL et al.** Flow Cytometry Bioinformatics. *PLoS Computational Biology*, 05 December 2013, vol. 9 (12)